# EUROPEAN PATENT APPLICATION

(11) **EP 4 606 787 A1**
(43) Date of publication of application: **27.08.2025**
(21) Application number: 24158806.0
(22) Date of filing: 21.02.2024
(51) Int. Cl.: C07C 209/62, C07C 209/84

(54) **METHOD FOR EFFICIENT PURIFICATION OF HEXAMETHYLENE DIAMINE OBTAINED FROM A RECYCLING PROCESS**

(71) Applicant: Covestro Deutschland AG, 51373 Leverkusen (DE)
(72) Inventor: Leimbrink, Mathias, 51373 Leverkusen (DE); Krause, Jonas, 51373 Leverkusen (DE); Altuntepe, Emrah, 51373 Leverkusen (DE); Bausa, Juergen, 51373 Leverkusen (DE); Waltermann, Thomas, 51373 Leverkusen (DE)
(74) Representative: Levpat

(57) **Abstract**

The present invention relates to a method for obtaining aqueous solutions containing hexamethylene diamine by depolymerizing polyamides and efficiently separating said hexamethylene diamine from the aqueous solution.

## Description

The present invention relates to a method for obtaining aqueous solutions containing hexamethylene diamine by depolymerizing polyamides and efficiently separating said hexamethylene diamine from the aqueous solution.

Hexamethylene diamine (HDA, HMDA, or hexane-1,6-diamine), is an important starting material in the production of polymers. It is used, for example, for the production of polyamides by polycondensing the diamine with dicarboxylic acids. Polyamide-6.6 (PA66, Nylon^{®}), for example, is generated from hexamethylene diamine and adipic acid. Another important use is the phosgenation of HMDA to the corresponding diisocyanate, which can then be converted in further steps into polyurethanes, polyureas or polyisocyanurates.

Currently, the use of HMDA in the manufacture of polymers such as polyamides, is a one way street: Once the polymer is manufactured, HMDA is captured therein. Conventional recycling processes attempt to re-use the HMDA-based polymer as such. Since polyamides are thermoplastic, this may be done by melting the product or by mechanical means. However, it may be desirable to split HMDA-based polymers in order to isolate the monomeric compounds once used in their synthesis. Such chemical recycling enhances the flexibility of recycling processes because the monomers do not remain "locked" in the polymer and may be used for the synthesis of different compounds. In the case of polyamides has been described this may be achieved e.g. by chemical hydrolysis as described e.g. by US 3,069,465, EP 0 833 809, EP 0 646 106, US 2,840,606 or US 5,302,756.

Aqueous solutions of diamines may also be the product of the production of these compounds by microbial fermentation. Recovering a diamine from the aqueous fermentation medium can be carried out as disclosed in EP 2684867 A1, for example, after most of the cell components have been removed. There, a method for the production of pentamethylene diamine is disclosed, in which the diamine is extracted in a liquid phase extraction process from the aqueous phase that has optionally been concentrated by distillation beforehand. As extractants, non-halogen aliphatic solvents, preferably straight chain alcohols with 4 to 7 carbon atoms, are recommended. These extractants must subsequently be separated from the product again by distillation. For the distillation processes to concentrate the aqueous raw product or to isolate the pentamethylene diamine from the extract, the use of one or more distillation columns and a pressure of between 0.1 kPa and normal pressure is described in each case.

US 20170369913 A1 discloses extraction of HMDA from aqueous solutions with various extractants and distillation of hexamethylene diamine. In this case the HMDA has been produced by biological means).

In order to improve the efficiency of HMDA-recycling, there is a need for processes for separating HMDA from an aqueous solution having improved energy efficiency. An improved energy efficiency decreases both economic costs and the carbon footprint of HMDA-recycling.

It is therefore the object of the present invention to provide a process for the separation of HMDA obtained from depolymerization processes from an aqueous solution having improved energy efficiency.

This object is solved by a method comprising the steps:
a) providing an aqueous solution containing hexamethylene diamine (HMDA) by depolymerizing an HMDA-based polymer,
b) extracting the HMDA-containing aqueous solution with a monovalent alcohol having 4 to 8 carbon atoms,
c) obtaining an HMDA-containing alcoholic phase, and
d) distilling the HMDA-containing alcoholic phase at a pressure of 100-600 mbar and separating the HMDA from the monovalent alcohol.

The HMDA present in the solution provided in method step a) is preferably present in uncharged form. This is typically the case at a pH of at least 11.5. Therefore, the HMDA-containing aqueous solution in method step a) preferably has a pH of at least 11.5, more preferably 12.0.

The monovalent alcohol may be selected from the group consisting of 1-pentanol, 1-hexanol, 1-heptanol or mixtures thereof.

In a preferred embodiment of the preset invention, the HMDA-containing alcoholic phase is distilled in step d) at a pressure of 150-400 mbar.

Further, the HMDA-containing alcoholic phase may be distilled in step d) with a single distillation column.

Moreover, step b) may be carried out using an extraction column and/or a mixer-settler, having 3 to 15 theoretical stages.

In accordance with the invention, the expressions "comprising", "including" or "containing" mean preferably "consisting substantially of" and particularly preferably "consisting of".

Statements concerning the content of organic compounds in the context of the present invention refer, unless stated otherwise, to values determined by gas chromatography. The skilled person is familiar with the quantitative evaluation of gas chromatograms, optionally using an internal standard.

"Extraction" in the present case is understood to mean a separation process consisting of the separation of a substance from a matrix, wherein in the present case substance and matrix are both liquids to result in a liquid-liquid extraction. Two immiscible phases are combined to separate a solute from one phase into the other, according to the relative solubility in each of the phases. Suitable extraction devices for the liquid-liquid extraction are well known in the art.

"Distillation" in the present case is understood to mean a thermal separation process used to recover evaporable substances, preferably liquids, from a composition. These separated vapors are subsequently precipitated, usually by condensation. The term encompasses in particular repeated evaporation and condensation using a column (distillation column) with a plurality of separating stages. Processes of this kind in which several distillation steps are arranged in a series in a column should strictly speaking be called rectification, but will nevertheless likewise be referred to herein as distillation for the purpose of simplification. The advantage of these processes is the powerful separating effect and the possibility of operating the plant continuously. A "distillation apparatus" is accordingly an apparatus which is suitable for performing a thermal separation process of this kind, i.e. for example sieve plate columns, packed columns, packed-bed columns, bubble cap tray columns or even single-stage evaporators such as, for example, falling film evaporators, thin-film evaporators, flash evaporators, multi-phase helical-coil evaporators, or natural or forced circulation evaporators.

The individual process steps will be explained in more detail below.

In the first step a) of the method of the invention, an HMDA-containing aqueous solution is provided by depolymerizing an HMDA-based polymer or a mixture of polymers containing an HDMA-based polymer. The polymer may be coated or may comprise an organic or inorganic filler. The content of HMDA can be freely chosen to allow efficient extraction of the HMDA. The aqueous composition comprises at least HMDA and water. Typically, it will comprise further depolymerization products of the polymer. In case of depolymerization of polyamides obtained from HMDA and a dicarboxylic acid, in particular polyamide 6.4, 6.6, 6.9, 6.10 and 6.12, such an additional depolymerization product may be the respective dicarboxylic acid. Further, the aqueous composition may comprise residues of the of catalysts and/or additives used for the depolymerization reaction, particularly salts. It may also comprise residues of any material which was used as a coating or filler of the original polymer.

In one preferred embodiment, the additional depolymerization product is separated from the aqueous composition in method step a) so that the composition present at the beginning of method step b) comprises HMDA, residues of the of catalysts and/or additives used for the depolymerization reaction but no further depolymerization products.

In a further preferred embodiment of the present invention, method step a) comprises a further sub-step of decreasing the water content of the composition after depolymerization. This sub-step may be performed in addition to the separation of additional depolymerization products or as an alternative thereto. A preferred method is the partial removal of water using a distillation as described in PCT/EP2022/075933.

Independently of the additional constituents of the aqueous composition comprising HMDA at the end of method step a) the composition comprises 0.1 to 50 wt.-% HMDA, preferably 5 to 30 wt.-% HMDA and most preferably 7 to 18 wt.-% HMDA. The water content of the composition is preferably at least 50 wt.-%, more preferably at least 55 wt.-%. All percentages are based on the total weight of the composition as 100 wt.-%.

The term "HMDA-based polymer" refers to all polymers that can release HMDA as single molecule using the appropriate chemical reactions. Depending on the type of polymer, additional compounds may be released as additional depolymerization products. For polyamides obtained by reacting HMDA and a dicarboxylic acid the additional depolymerization product will be the respective dicarboxylic acid, its salt or a derivative of this acid. Preferred "HMDA-based polymers" are polyamides and polyurethanes. Preferred polyamides are polyamide 6.4, polyamide 6.6, polyamide 6.9 and polyamide 6.10. A particularly preferred "HMDA-based polymer" is polyamide 6.6 which is the reaction product of adipic acid and HMDA.

The appropriate chemical reaction which releases HMDA from a polymer obviously depends on the type of bonds present in the polymer. In case of polyamides such chemical reaction needs to cleave the amide bond so that HMDA is released. In case of polyamides, it is preferred to cleave the amide bond by hydrolysis

Preferred methods for the hydrolysis of polyamides are ammonolysis, acidic hydrolysis, alkaline hydrolysis, glycolysis, hydrolysis by microwaves and enzymatic hydrolysis. Amongst these acidic and alkaline hydrolysis are preferred with particular preference for alkaline hydrolysis.

Preferred agents for acidic hydrolysis are aqueous solutions ofHCl, formic acid, sulphuric acid and nitric acid. Suitable reaction temperatures for acidic hydrolysis are in the range between 80 and 120 °C. Acidic hydrolysis is preferably performed at a pressure between 1 and 6 bar. Suitable methods for acidic hydrolysis are described in US 3,069,465 and EP 0 833 809.

Preferred agents for alkaline hydrolysis are alkali hydroxide solutions in water or mixtures of water and alcohols. Preferred alkali hydroxides are NaOH and KOH. Particularly preferred are aqueous solutions of NaOH and KOH. Suitable reaction temperatures for alkaline hydrolysis are in the range between 140 and 220 °C. Alkaline hydrolysis is preferably performed at a pressure between 0.8 and 150 bar. Suitable methods for alkaline hydrolysis are described in EP 0 646 106 or US 2,840,606.

Ammonolysis is preferably performed at temperatures between 300 and 350 °C and pressures between 34 and 172 bar. A suitable method is described in US 5,302,756.

It is to be understood that method steps a) and b) may be performed at different locations and/or with a time lag. In one preferred embodiment of the present invention, the distance between the location, where method step b) is performed and the location, where method step b) is performed, is at least 1 km, preferably at least 5 km, more preferably at least 10 km and most preferably at least 20 km. In another preferred embodiment of the present invention, the time lag between the end of method step a) and the commencement of method step b) is at least 1 hour, preferably at least 12 hours, more preferably at least 24 hours and most preferably at least 72 hours.

In certain embodiments of the present invention, method step a) may be preceded by a method step by breaking a polymer part into smaller particles so that the surface to volume ratio of the polymer increases, thus facilitating depolymerization. This can be achieved by all suitable methods known to person skilled in the art.

In the second step b) of the method of the invention, the HMDA-containing aqueous solution provided in step a) is extracted with a monovalent alcohol having 4 to 8 carbon atoms.

The monovalent alcohol must enable an efficient extraction of the HMDA. If too much water is carried over, the energy consumption of the distillation increases. Further, the monovalent alcohol must also be able to carry a high concentration of HMDA, so that the amount of alcohol required for the extraction of the HMDA is minimized as distillation of the alcohol requires energy.

The monovalent alcohol is not particularly restricted as long as it has 4 to 8 carbon atoms, and the monovalent alcohol may include all possible isomers. The monovalent alcohol may be selected from n-butanol, i-butanol, sec-butanol, 1-pentanol, 2-pentanol, 3-pentanol, neopentyl alcohol, 1-hexanol, 2-hexanol, 3-hexanol, 1-heptanol, 2-heptanol, 3-heptanol, 4-heptanol, 1-octanol, 2-octanol, 3-octanol, 4-octanol, cyclopentanol, cyclohexanol, cycloheptanol or cyclooctanol or mixtures thereof. In a preferred embodiment of the present invention the monovalent alcohol may be selected from 1-pentanol, 1-hexanol, 1-heptanol or mixtures thereof. In a more preferred embodiment of the present invention the monovalent alcohol may be 1-hexanol.

Step b) may be carried out by any suitable extracting device, which is known in the art. Extraction may be carried out using an extraction column and/or a mixture-settler, having 3 to 15 theoretical stages.

Extraction conditions are not limited, the extraction may be carried out at room temperature (20-25°C) and/or under ambient pressure. The higher the temperature the higher the partition coefficient, thus extraction efficiency is increased. The extraction temperature is limited by the boiling temperature of water or the monovalent alcohol used as extractant. The extraction may be carried out under ambient pressure. A phase ratio, i.e., mass ratio, of alcohol: water is preferably from 0.1 to 1, more preferably from 0.2 to 0.5 and most preferably from 0.3 to 0.4.

In the third step c) of the method of the invention, an HMDA-containing alcoholic phase is obtained. This HMDA-containing alcoholic phase is the organic phase obtained after extraction in step b). The other phase is the aqueous phase. As HMDA has a higher solubility in the monovalent alcohol than in water, the HMDA will be concentrated in the organic alcoholic phase. The HMDA-containing alcoholic phase may contain, besides HMDA and monovalent alcohol, also residual amounts of water.

The HMDA-containing alcoholic phase (organic phase) may be easily separated from the aqueous phase after extraction by means well known in the art.

In the fourth step d) of the method of the invention, the HMDA-containing alcoholic phase obtained in step c) is distilled at a pressure of 100-600 mbar, and, thus, the HMDA is separated from the monovalent alcohol and the optionally residual amounts of water.

It was surprisingly found that the efficiency of the distillation step d) depends on the applied pressure as the equilibrium of vapor and liquid phases is highly pressure-dependent.

Best distillation results are achieved within a pressure optimum for the distillation step.

A distillation device used in step d) is not limited, but any distillation device known in the art for such separation steps may be used. The distillation device may be a single distillation column. The single distillation column may have 5 to 100, 10 to 60 or 20 to 50 theoretical stages. In case, 1-hexanol is the monovalent alcohol, 20 or more theoretical stages, e.g., 20 to 50 theoretical stages, may be used. The distillation pressure may be from 150-400 mbar. It is, thus, lower than the atmospheric pressure of 1,013 mbar.

In another preferred embodiment of the present invention, the distillation pressure is 150 to 400 mbar, preferably 200-400 mbar, and the monovalent alcohol is be 1-hexanol. The distillation pressure is 200-400 mbar, preferably 150 to 400 mbar, and the monovalent alcohol is 1-butanol.

Typically, during the distillation, the monovalent alcohol used has a lower boiling point than the HMDA and is evaporated to the top of the distillation device, may be condensed and may then be removed from the distillation device and may be optionally reused as extractant. HMDA typically has the higher boiling point and is, thus, concentrated at the bottom of the distillation device and may be removed therefrom for further processing.

The HMDA-containing aqueous solution may contain substances having higher or lower boiling points compared to HMDA.

Substances with lower boiling points compared to HMDA are, for example, apart from water, ammonia, etc. In addition, gases such as nitrogen or carbon dioxide may be present in the composition in dissolved or bound form.

Substances with higher boiling points compared to HMDA can occur.

### Brief description of the drawings

Fig. 1 is a graph illustrating the dependency of the ideal separation factor on temperature for a mixture of C6-diamine (HMDA) and different monovalent alcohols;
Fig. 2 is a graph illustrating the dependency of the ideal separation factor on the temperature for a mixture of C6-monoamine (hexaneamine) and different monovalent alcohols;
Fig. 3 is a graph illustrating the dependency of the ideal separation factor on the temperature for a mixture of a C6-diamine (HMDA) and different divalent alcohols;
Fig. 4 is a graph illustrating the dependency of the ideal separation factor on the temperature for a mixture of a C6-monoamine (hexaneamine) and different divalent alcohols;
Fig. 5 is a graph illustrating vapor-liquid equilibrium (VLE) data for a mixture of 1-hexanol and hexamethylene diamine; experimental data (data points) was generated with a circulating still - an isobaric dynamic method (here only data at 1000 mbar is shown). The ideal VLE of 1-hexanol and hexamethylene diamine was calculated and an UNIFAC estimation was performed, as well. Experimental data was used to regress NRTL parameters. The calculation with NRTL using these parameters is also shown.
Fig. 6 is a graph illustrating the effect of operating pressure on energy demand in a distillation step of the present invention. It is the distillation of HMDA/1-hexanol. The temperature during distillation is dependent on the pressure and is not constant. The heat duty is the totally required heat input. The graph shows a calculated example using the software Aspentech Aspen plus based on parameters derived from the illustrated measurements.

### Examples

The present invention is further illustrated by means of examples and comparative examples. The examples shall not restrict the scope of protection which is solely defined by the claims.

In the inventive method, step d) of distilling the HMDA-containing alcoholic phase is the most essential step. To illustrate that advantageous effects may be achieved when distilling an appropriately selected combination of diamine and monovalent alcohol, several combinations of monovalent and divalent alcohols with mono- and diamines, respectively, were investigated. All vapor pressures of pure components are reported in literature and available data was used.

Four different types of combinations were investigated, as follows:
1. monovalent alcohol + diamine
2. monovalent alcohol + monovalent amine
3. divalent alcohol + diamine
4. divalent alcohol + monovalent amine

The combination of monovalent alcohol + diamine is according to the invention, the other combinations are comparative examples.

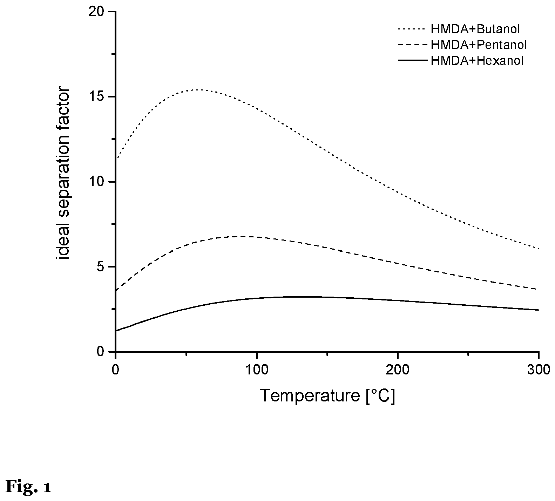

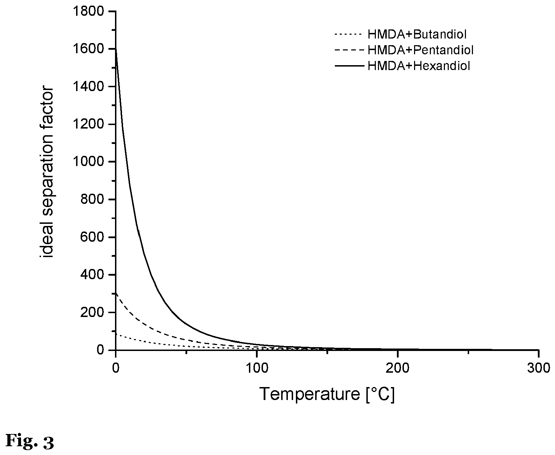

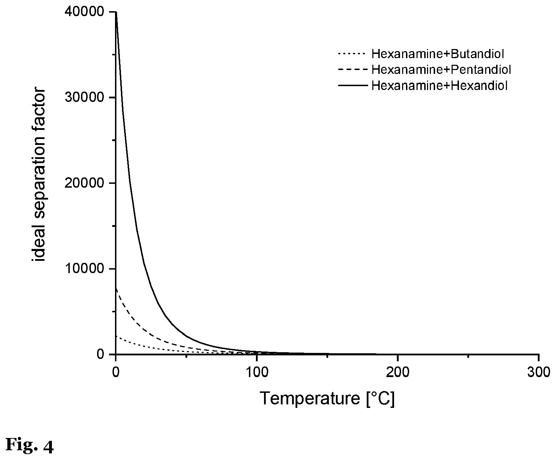

In the examples, hexanediamine was used as diamine and hexanamine was used as monoamine. The same findings as shown in Fig. 1-4 account also for C4 amines and diamines. C4 to C6 monovalent and divalent alcohols are investigated in these studies as given in Fig. 1-4.

(Ideal) relative volatilityFor each example and comparative example of Fig. 1-4, a mixture of amine and alcohol was used to calculate, based on literature values, the (ideal) relative volatility (also referred to as separation factor) which is the ratio of the vapor pressures of the pure compounds of amine and alcohol at a given temperature, where the vapor pressure of the low-boiler is divided by the vapor pressure of the high-boiler.

The separation factors for the respective mixtures are summarized in Fig. 1-4.

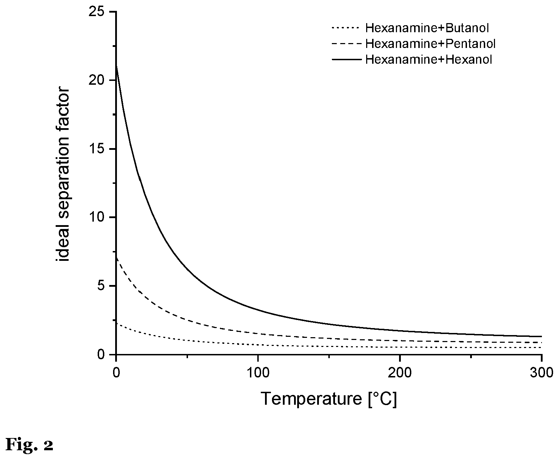

From Fig. 1-4 it can be seen that the combinations of monovalent alcohols and diamines appear to behave in a totally unexpected manner. From basic textbook knowledge and also from the behavior of similar combinations (Fig. 2 to 4) it is always assumed that a separation gets easier with lowering the pressure (separation factor increases steadily with lowering the pressure - corresponds to lowering the temperature). But the separation factor of mixtures of monovalent alcohols and hexanediamine shows a specific maximum (this also accounts for putrescine in mixtures with C4 to C6 monovalent alcohols).

### Vapor-liquid equilibrium

To understand the real behavior of the mixtures, experimental vapor-liquid equilibrium (VLE) data have been measured for monovalent alcohol and hexanediamine mixtures. These were binary isobaric VLE measurements of the monovalent alcohols 1-butanol, 1-pentanol and 1-hexanol in combination with hexanediamine. The measurements of each binary VLE have been performed for three different pressures.

In summary, 9 experiments have been performed. These were conducted in a dynamic circulation still (ebulliometer as a standard method well known in the art) with sampling of condensed vapor phase and liquid phase followed by a gas-chromatographic quantification of alcohol and diamine. The pressure in the cell was held constant and boiling temperatures of mixtures with different concentrations were measured.

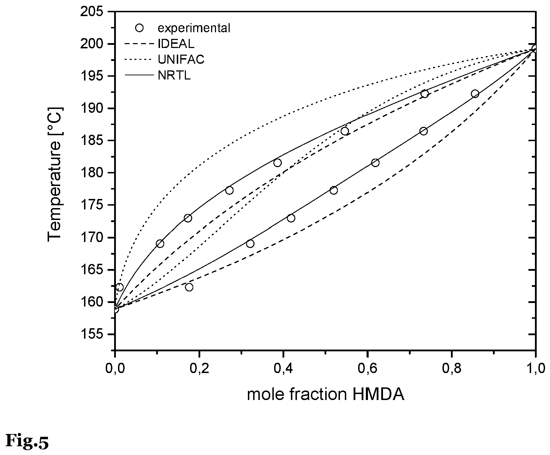

In Fig.5, the measured VLE of 1-hexanol and hexanediamine at 1000 mbar is shown exemplarily. Experimental data is compared to both an ideal VLE determination and an VLE estimation with a predictive thermodynamic model named UNIFAC ("Universal Quasichemical Functional Group Activity Coefficients").

In Fig. 5, points indicate experimental data, solid line is calculation with NRTL model (with regressed parameters), dashed line is an ideal calculation, dotted line is a prediction with UNIFAC estimation method ("Universal Quasichemical Functional Group Activity Coefficients", known in the art). In Fig. 5, an ideal calculation of the phase equilibrium and an UNIFAC estimation is included. It can be clearly seen that the experimental data (data points) deviate from both of these. This makes it obvious that without measurements, the real behavior of the systems could not be predicted properly.

Model parameters of the NRTL ("non-random-two-liquid", known in the art) model for all investigated systems have been regressed to the measured data (solid line in Fig. 5). It can be stated that the model fits nicely to the data. The regressed parameters have been used in all simulation studies from which insights regarding the process have been retrieved. This means that all simulation studies are based on experimental VLE data for several monovalent alcohol+hexanediamine experiments at different pressures.

### Effect of operating pressure on energy demand

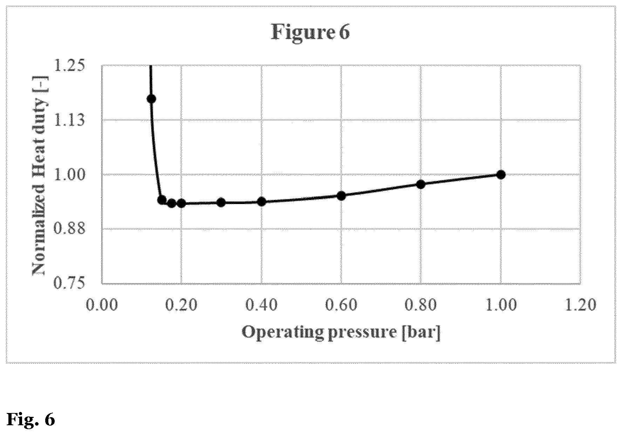

The effect of the distillation column operating pressure on the energy demand of the separation was investigated in more depth using a rigorous process model based on the experimentally regressed NRTL parameters. For the exemplary mixture of 1-hexanol and hexanediamine used before (see Fig. 5), the influence of the operating pressure on the energy demand of separation is depicted in Fig. 6. For the simulations, the composition of the mixture to be separated is representative for the extract phase from the previous liquid-liquid-extraction step and also includes water present due to cross solubility in 1-hexanol as the exemplary solvent. The energy demand for the distillative separation of hexanediamine from 1-hexanol and water shows a significant dependency on the applied column pressure. By decreasing the column pressure from atmospheric pressure to vacuum, the heat duty for the distillation decreased and thus, the separation becomes more energy efficient. For operating pressure between 200 to 400 mbar, an optimum in the applied pressure is found. However, when the pressure is decreased further, due to the thermodynamic behavior of the binary system 1-hexanol / hexanediamine the energy demand for the separation starts to increase significantly. This significant increase is directly related to the non-ideal behavior of the mixture and could be quantified based on the experimental VLE measurements performed for the different pressure levels as shown exemplarily in Fig. 5.

The features disclosed in the foregoing description, the claims as well as the drawing may, both separately and in any combination thereof, be material for realizing the aspects of the disclosure made in the independent claims, in diverse forms thereof.

## Claims

1. Method for separating HMDA from an aqueous solution, comprising the steps:
a) providing an aqueous solution containing hexamethylene diamine (HMDA) by depolymerizing an HMDA-based polymer,
b) extracting the HMDA-containing aqueous solution with a monovalent alcohol having 4 to 8 carbon atoms,
c) obtaining an HMDA-containing alcoholic phase, and
d) distilling the HMDA-containing alcoholic phase at a pressure of 100-600 mbar and separating the HMDA from the monovalent alcohol.

2. The method of claim 1, wherein method step d) is performed at a pressure of 150 to 400 mbar.

3. The method according to claim 1 or 2, wherein the monovalent alcohol is selected from the group consisting of 1-pentanol, 1-hexanol, 1-heptanol or mixtures thereof.

4. The method according to any of the preceding claims, wherein the HMDA-containing alcoholic phase is distilled in step d) with a single distillation column.

5. The method according to any of the preceding claims, wherein step b) is carried out using an extraction column and/or a mixer-settler, having 3 to 15 theoretical stages.

6. The method according to any of the preceding claims, wherein the HMDA-based polymer is a polyamide obtained by (i) condensation of HMDA and a dicarboxylic acid or (ii) a polyurethane.

7. The method according to claim 6, wherein the polyamide is selected from the group consisting of polyamide 6.4, polyamide 6.6, polyamide 6.9 polyamide 6.10 and polyamide 6.12.

8. The method according to claim 6 or 7, wherein depolymerization of the polyamide is achieved by a method selected from the group consisting of acidic hydrolysis, alkaline hydrolysis, ammonolysis, glycolysis, hydrolysis by microwaves and enzymatic hydrolysis

9. The method of any one of the preceding claims, wherein method step a) and method step b) are performed at different locations with a minimum distance of 1 km.
